Europäisches Patentamt

European Patent Office

Office européen des brevets

(19)

(11) Publication number: **0 070 719**
**B1**

# EUROPEAN PATENT SPECIFICATION

(45) Date of publication of patent specification: **11.12.85**

(21) Application number: **82303766.8**

(22) Date of filing: **19.07.82**

(51) Int. Cl.⁴: **B 01 J 13/02,** A 23 L 1/22,
A 61 K 7/00, A 61 K 7/46,
A 61 K 9/50, A 23 P 1/00

(54) **Encapsulation of volatile liquids.**

(30) Priority: **21.07.81 GB 8122414**

(43) Date of publication of application:
**26.01.83 Bulletin 83/04**

(45) Publication of the grant of the patent:
**11.12.85 Bulletin 85/50**

(84) Designated Contracting States:
**AT BE CH DE FR GB IT LI NL SE**

(56) References cited:
**EP-A-0 011 324**
**US-A-3 159 585**
**US-A-3 903 295**

(73) Proprietor: **UNILEVER PLC**
**Unilever House Blackfriars P.O. Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(72) Inventor: **Johnson, Richard Shaw**
**24 Woodlands Road**
**Irby Wirral Merseyside (GB)**

(74) Representative: **Farndon, John Ernest et al**
**UNILEVER PLC Patents Division P.O. Box 68**
**Unilever House**
**London EC4P 4BQ (GB)**

Courier Press, Leamington Spa, England.

## Description

This invention relates to the encapsulation of volatile liquids. More particularly, the invention is concerned with the preparation of encapsulates of volatile liquids which are of an adequate size for their particular end-use. Such uses can include their introduction into various powdered compositions where, due to their size, they do not suffer segregation problems in relation to the rest of the composition. Likewise, they can be chosen to be of such a size that they do not reduce the flow properties of powdered compositions into which they are incorporated.

It has previously been proposed to prepare encapsulates of volatile liquids such as volatile natural or synthetic flavouring oils and perfumes in various carrier materials such as starch and dextrin derivatives by spray-drying emulsions containing the carrier material and volatile liquids (see, for example, United States Patent Specification No. 3 159 585).

European Patent Application 0 011 324 discloses a fluidised bed process for making beverages, food and the like which, for example, involves spraying small drops of aroma/flavour concentrates into a fluidised bed of dry coffee solids in a process in which each atomised droplet is coated with dry coffee solids to lock in desirable aromas and flavours.

US Patent No. 3 903 295 discloses a process for encapsulating a material which tends to deteriorate upon exposure to an auto-oxidative, humid or thermal atmosphere, by forming the material in a viscid or gel-like medium and dispersing the medium as particulates having a tacky consistency in an environment containing an agitated quantity of a powdered, sorbent, film-forming agent such as a gum, carboxymethyl cellulose, or ethyl cellulose, so as to form an encapsulating, substantially non-friable film around each of said particulates and recovering the encapsulated particulates.

However, in any spray-drying equipment, the particle size of the product from the spray-drying tower is thought to be related to the possible residence time of the droplets in the tower and of the ability of the particles to agglomerate and if large particles are required, a large tower must be used, which will inherently mean the production of large quantities of encapsulate material. In addition the viscosity characteristics of some carrier materials in emulsion or solution form can cause problems in atomisation in spray-drying towers.

Attempts have been made to obtain larger particles by the agglomeration of spray-dried particles in an agglomerator. However, when using a pan granulator or fluidised bed agglomeration to increase the size of spray-dried particles containing a volatile liquid, significant losses of that liquid are found to occur either during the agglomeration process or in subsequent storage, probably as a result of changes in the particle structure and their effect on its retention of the volatile liquid.

For various uses in the flavour and perfumery field, relatively small quantities of very specialised encapsulates are required and it is desirable that these encapsulates are of a particle size greater than that which can be achieved using small pilot plant spray-drying towers.

For example, it is desirable to introduce into fabric detergent washing powders encapsulates containing perfumes and it is difficult, using normal, small spray-drying equipment, to produce such encapsulate particles greater than 100 microns diameter. Ideally, for use in a fabric detergent powder, an encapsulate would be of the order of 500 microns diameter and such particles cannot be produced except in very large spray-drying towers of the size used in producing the detergent powder itself.

Similar criteria are involved in producing flavour-containing encapsulate particles for use in the foods industry and the present invention provides a process for the production of encapsulates of substantially greater size than those which can be made in small spray-drying towers which do not generate powder flow problems and which can readily be re-dissolved in, for example, water, without lumping or clustering together and so slowing down their solution.

Accordingly, the present invention provides a process for the encapsulation of a volatile liquid in a carrier material, in which a solution of the volatile liquid is sprayed onto particulate solid material characterised in that an aqueous emulsion of the volatile liquid in an aqueous solution of the carrier material containing at least 25% by weight of the carrier material is agglomerated in a fluidised bed by spraying the emulsion onto particles which are either particulate carrier material or encapsulates containing the volatile liquid.

The volatile liquid used in this invention may be a flavouring oil or a perfume blend. It will be understood that such volatile liquids can contain, in solution, the usual gums, resins and vegetable and animal extracts normally used in the flavour and perfumery industries. It has been surprisingly found that even the very volatile "top notes" of perfumes can usefully be encapsulated using this process. This is particularly surprising, since in a paper read at the 2nd International Conference on Powder Technology (Powtech Conference) in 1975 in England, in a paper entitled "Fluidised Bed Processing of Bulk Solids" by E. J. Simon of Aeromatic Limited, Muttenz, Switzerland, the manufacturers of one fluidised bed system suggested that "The big advantage of coating in the fluid bed is that the solvents being used are immediately vaporised in the hot air stream, they cannot penetrate into the kernels and unexpected side-effects can be easily avoided".

It is not fully understood why this benefit has now been found in this equipment, but it may be related to the selection of the carrier material.

Suitable carrier materials for use in this invention include modified starches, such as dextrinised, acid-thinned and oxidised starches and maltodextrin. A preferred carrier is a dextrinised starch containing controlled amounts of hydrophobic side groups sold by Laing-National Limited in the United Kingdom, under the Trade Name "Capsul". Other suitable carrier materials, again, depending on the end of the encapsulate, include gums such as gum-acacia and gelatins.

In general, it is desirable to use, as the carrier material, a material of adequate water solubility, such that a solution of at least 25% by weight solids can be readily achieved, preferably at least 35% by weight solids in water. It is also desirable that the flow characteristics of the aqueous solution are such that it can readily be sprayed.

Using the process provided by this invention it is possible to obtain a volatile liquid content in the carrier exceeding 30% by weight with a retention of over 90%.

A further important benefit arising from this process is that when the volatile liquid was a perfume, experienced perfumers were satisfied that the odour characteristics of the encapsulated perfume had not changed significantly from those of the original perfume.

The carrier concentration in water is conveniently in the range 30—50% by weight and the volatile liquid content in the emulsion is up to 50% by weight.

The fluidised bed apparatus for use in this process can be selected from those of various manufacturers, including Aeromatic AG of Muttenz in Switzerland and "Strea-1" laboratory agglomerator, also supplied by Aeromatic AG. Other useful agglomerators are supplied by the Calmic Division of William Boulton of Burslem, England.

The process may be carried out by spraying an emulsion of volatile liquid and a suitable carrier material in water into the fluidised bed agglomerator, which has previously been charged with a small quantity of a particulate carrier material or, preferably, with a sample of small encapsulates containing the volatile liquid to be employed in the bulk preparation.

The particles onto which the emulsion is sprayed can also be particulate material selected from edible materials such as powdered tea and ground spices or herbs.

The emulsion containing the volatile liquid coats the particulate material fluidised by the passage of air through the bed and causes some agglomeration of the particles and a build-up of the components of the emulsion including the volatile liquid. Since the residence time in the fluidised bed is controllable, the spraying of the emulsion may be continued until the required particle size of encapsulate has been obtained.

In commercial practice, it is desirable to run the process as a continuous one, using equipment of appropriate size for the production required. Such continuous running tends to ensure the maximum uniformity of product. To achieve this, careful control of input spray rate, fluidisation air-flow rate and its temperature must be exercised.

Furthermore, since the residence time is not critical, the equipment used for spraying in the emulsion onto the bed may be chosen to cover a wider range of viscosity and flow characteristics than is normally available in a spray-drying tower.

Details of actual experimental runs and the experimental conditions under which the agglomerator was used are set out in the following example.

Example 1

This example describes the preparation of an encapsulate containing a nominal 30% w/w of a volatile perfume, a "top note" composition in a "Strea-1" laboratory agglomerator using corn starch as the initial support material.

Preparation of emulsion

400 g of Capsul, a partially dextrinised chemically modified starch available from Laing-National Limited, was dissolved with agitation in 600 g of water held at 70—80°C over a period of 20 minutes. This solution was then cooled and 250 g of a perfume "top note" composition (LP 843) was added, the mixture being vigorously agitated by means of a "Silverson" high-speed homogeniser. As a result the perfume was completely emulsified within the aqueous phase, the emulsion being stable for at least 24 hours.

Encapsulation

100 g of corn starch of particle size of about 20 microns was introduced in to the chamber of a "Strea-1" laboratory agglomerator (suitable for production of 1 Kg of product) and the chamber, fitted with filters and spray-nozzle, was placed in position on the machine. The corn starch was then fluidised with air heated to an inlet temperature of 80—100°C. The degree of fluidisation could be controlled by varying the air flow rate and typically was adjusted so that the maximum lift of the corn starch particles within the chamber was below the position of the spray-nozzle. The emulsion was then pumped into the agglomerator by means of a Watson-Marlow Limited peristaltic pump and atomised at the two-fluid spray-nozzle, atomisation being effected by air at a pressure of between 1 and 2 bar. The rate of pumping of the emulsion was such that the bed remained free-flowing and fluidised throughout the preparation. Typically an emulsion flow rate of between 10 and 20 g/min was used. Fluidisation could also be controlled by adjustments to the fluidising air flow rate, some increase during the agglomeration generally being necessary. Typical machine parameters during a preparation are as follows:
inlet temp. 100°C
    Air inlet temp. 100°C
    Air outlet temp. 55°C
    Fluidising air control setting 4 Atomiser nozzle air pressure 1.5 bar
Filter resistance 100 mm H$_2$O
Support plate resistance 60 mm H$_2$O
Air flow rate 50 m³/hr
When all of the emulsion had been added fluidisation was continued for 2 to 3 minutes then stopped and the product discharged from the agglomerating chamber.

A typical product analysis was as follows:
Perfume content 29.0%, i.e. 96.7% of nominal.
Overall yield 97%.

Particle size distribution (calculated according to Rosin-Rammler parameters, see Journal Institute of Fuel, October 1933, pages 29—36) diameter=730 μ n=2.1.

The "top note" formulation LP 843 consisted of:

|  | % by weight |
|---|---|
| Lavandin abrialis | 40.0 |
| Eucalyptus globulus | 10.0 |
| Rosemary Spanish | 5.0 |
| Ethyl amyl ketone | 2.0 |
| Hexyl acetate | 1.0 |
| Methyl hexyl ether | 0.5 |
| Turpentine | 5.0 |
| Linalyl acetate | 10.0 |
| Linalool | 10.0 |
| Ocimene | 3.0 |
| Camphor powder synthetic | 4.5 |
| Borneol | 1.5 |
| Terpinyl acetate | 7.5 |
|  | 100.0 |

A sample of the encapsulate prepared according to this Example was placed in water and, when compared by a perfumer with the original LP 843 formulation, was considered to have produce an acceptably similar note.

Example 2

This example describes the use of an Aeromatic AES 5.5 production size agglomerator having a nominal capacity of 15 Kg. The controls on this machine were essentially identical with those of the "Strea-1" described in Example 1.

10 Kg of emulsion containing a lemon perfume were prepared by the same procedure as described in Example 1. The support material comprised 2 Kg of agglomerate containing this lemon perfume prepared as described in Example 1 and contained 29.2% w/w of the perfume composition and had a particle size distribution defined by diameter=860 μ n=2.3.

The support material was placed in the agglomerator and fluidised and the emulsion sprayed in via a "Schlick" two fluid nozzle operated at 2.5 bar air pressure. The air inlet temperature was 95°C. The emulsion addition rate was initially 2.5 Kg/hr being progressively increased during the first half-hour to a maximum of 5.6 Kg/hr as the bed built up. After a period of just under 2 hours the agglomerating chamber was full and spraying of the emulsion was stopped. On discharge the total product was found to be 8.2 Kg, i.e. just over half the nominal machine capacity, and this represented an overall product yield of 97.4%. Product perfume content was 28.1% w/w and moisture content 3.1%. Particle size analysis gave diameter=820 μn=1.9, bulk density 0.38 g/cc.

A sample of the encapsulate prepared according to this Example was placed in water and, when compared by a perfumer with the original lemon perfume, was considered to have produced an acceptably similar note.

The perfume containing encapsulates provided by this invention can usefully be incorporated into detergent compositions and the perfume is protected against the action of various components of the detergent and also against humidity changes in storage.

Accordingly this invention also provides encapsulates made by the process of this invention. In addition, this invention provides a perfumed detergent composition comprising by weight:
(a) from 0.1% to 30% of a water-soluble organic surfactant;
(b) from 0.04% to 5% of encapsulate provided by this invention containing, as the volatile liquid, a perfume; and the remainder,
(c) detergency fillers and extenders.

Example 3

Using the apparatus and carrier material of Example 1, a flavour encapsulate was made with 250 g of lemon oil (FIL Kent, England) in place of the perfume. This encapsulate was found to have an improved shelf-like, when compared with flavour granules prepared from spray-dried flavour powders using the same lemon oil, and a more attractive organoleptic reception when tasted by a test panel.

Accordingly, this invention also provides an edible composition comprising an edible base and an organoleptically effective amount of an encapsulate provided by this invention in which the volatile liquid is a flavour.

Example 4

Using the apparatus and procedures of Example 1, run-flavoured granules were prepared. Gum acacia was employed both as carrier and initial particulate charge for the fluidised bed. The emulsion of flavour and carrier comprised, by weight:

94.82  parts of 40% aqueous gum acacia solution
0.554  part rum ether
0.741  parts vanalin
3.885  parts rum base reference 3683064 from PPF International Limited of Ashford, Kent, England.

The emulsion wus sprayed onto the powdered gum acacia in the fluidised bed and the final product met a specification of less than 2% over 2 mm aperture mesh and less than 2% less than 0.25 mm aperture mesh.

The rum flavoured granules were incorporated into teabags and stored for 6 months. Beverages prepared from the stored and freshly-prepared granules and tea compared satisfactorily in the view of an organoleptic panel.

Example 5

Example 4 was repeated using tea powder in place of the gum acacia powder as the initial particulate charge. A product very similar to that in Example 4 was obtained and it also had a satisfactory flavour after 6 months' storage.

**Claims**

1. A process for the encapsulation of a volatile liquid in a carrier material, in which a solution of the volatile liquid is sprayed onto particular solid material characterised in that an aqueous emulsion of the volatile liquid in an aqueous solution of the carrier material containing at least 25% by weight of the carrier material is agglomerated in a fluidised bed by spraying the emulsion onto particles which are either particulate carrier material or encapsulates containing the volatile liquid.

2. process as claimed in claim 1, in which the carrier material is selected from modified starches, dextrinised starches, acid-thinned starches, oxidised starches, dextrinised starches containing controlled amounts of hydrophobic groups, gums, including gum-acacia and gelatins.

3. A process as claimed in claim 1 or claim 2 in which the carrier material has a solubility sufficient to permit the preparation of solutions containing at least 35% solids which are sprayable.

4. A process as claimed in claim 1, 2 or 3, in which the volatile liquid is a perfume composition.

5. A perfumed detergent comprising by weight:
(a) from 0.1% to 30% of a water-soluble organic surfactant;
(b) from 0.04% to 5% of an encapsulate obtained by the process of claim 4; and the remainder,
(c) detergent fillers and extenders.

6. A process as claimed in claim 1, 2 or 3, in which the particles initially charged in the fluidised bed are selected from powdered tea, ground spices and ground herbs.

7. A process as claimed in claim 1, 2 or 3, or claim 6, in which the volatile liquid is a flavor composition.

8. An edible composition comprising an edible base and an organoleptically effective amount of an encapsulate obtained by the process of claim 7.

**Revendications**

1. Procédé pour encapsuler un liquide volatil dans un produit porteur, procédé dans lequel on vaporise une solution du liquide volatil sur un produit solide en particules, caractérisé en ce qu'une émulsion aqueuse du produit liquide volatil dans une solution aqueuse du produit porteur contenant au moins 25% en poids du produit porteur est agglomérée dans un lit fluidisé par pulvérisation de l'émulsion sur les particules qui sont soit du produit porteur en particules, soit des particules encapsulées contenant le liquide volatil.

2. Procédé comme revendiqué dans la revendication 1, dans lequel le produit porteur est choisi parmi des amidons modifiés, des amidons dextrinisés, des amidons dilués dans l'acide, des amidons oxydés, des amidons dextrinisés contentant des quantités contrôlées des groupes hydrophobes, des gommes, y compris de la gomme arabique et des gélatines.

3. Procédé comme revendiqué dans la revendication 1 ou la revendication 2, dans lequel le produit support a une solubilité suffisante pour permettre la préparation de solutions contenant au moins 35% de produit solide que l'on puisse pulvériser.

4. Procédé comme revendiqué dans la revendication 1, 2 ou 3, dans lequel le liquide volatil est une composition de parfum.

5. Composition détergente parfumée comportant en poids:
a) de 0,1% à 30% d'un produit tensioactif organique soluble dans l'eau;
b) de 0,04% à 5% d'un produit encapsulé obtenu par le procédé de la revendication 4; et le reste,
c) des charges détergentes et des diluants.

6. Procédé comme revendiqué dans la revendication 1, 2 ou 3, dans lequel les particules initialement chargées dans le lit fluidisé sont choisies parmi le thé en poudre, les épices et les herbes.

7. Procédé comme revendiqué dans la revendication 1, 2 ou 3 ou la revendication 6 dans lequel le liquide volatil est une composition d'arômes.

8. Composition comestible comportant une base comestible et une quantité efficace au point de vue organoleptique de particules encapsulées obtenues par le procédé de la revendication 7.

**Patentansprüche**

1. Verfahren zum Einkapseln einer flüchtigen Flüssigkeit in einem Trägermaterial, bei dem eine Lösung der flüchtigen Flüssigkeit auf teilchenförmiges festes Material gesprüht wird, dadurch gekennzeichnet, daß eine wässrige Emulsion der flüchtigen Flüssigkeit in einer wässrigen Lösung des Trägermaterials, die wenigstens 25 Gew.-% des Trägermaterials enthält, in einem Fließbett durch Aufsprühen der Emulsion auf Teilchen, die entweder teilchenförmiges Trägermaterial oder die flüchtige Flüssigkeit enthaltende Einkapselungen sind, agglomeriert wird.

2. Verfahren nach Anspruch 1, bei dem das Trägermaterial unter modifizierten Stärken, dextrinierten Stärken, säureverdünnten Stärken, oxidierten Stärken, dextrinierten Stärken, die gesteuerte Mengen hydrophober Gruppen enthalten, Gums einschließlich Gummi arabicum und Gelatinen ausgewählt wird.

3. Verfahren nach Anspruch 1 oder Anspruch 2, bei dem das Trägermaterial eine Löslichkeit hat, die ausreicht, die Herstellung von Lösungen, welche wenigstens 35% Feststoffe enthalten, die versprühbar sind, zu erlauben.

4. Verfahren nach Anspruch 1, 2 oder 3, bei dem die flüchtige Flüssigkeit eine Parfumkomposition ist.

5. Parfumierte Detergenszusammensetzung, umfassend gewichtsmäßig:
   (a) 0,1% bis 30% eines wasserlöslichen organischen Tensids;
   (b) 0,04 bis 5% einer nach dem Verfahren von Anspruch 4 erhatenen Einkapselung, und Rest
   (c) Detergens-Flüllstoffe und -Streckmittel.

6. Verfahren nach Anspruch 1, 2 oder 3, bei dem die anfangs in das Fließbett eingebrachten Teilchen unter gepulvertem Tee, zerkleinerten Gewürzen und zerkleinerten Kräutern ausgewählt werden.

7. Verfahren nach Anspruch 1, 2 oder 3 oder Anspruch 6, bei dem die flüchtige Flüssigkeit eine Aromazusammensetzung ist.

8. Verzehrbare Zusammensetzung, umfassend eine verzehrbare Grundlage une eine organoleptisch wirksame Menge einer nach dem Verfahren von Beispiel 7 erhaltenen Einkapselung.